# EUROPEAN PATENT APPLICATION

(11) **EP 4 494 635 A1**
(43) Date of publication of application: **22.01.2025**
(21) Application number: 24188907.0
(22) Date of filing: 16.07.2024
(51) Int. Cl.: A61K 9/14, A61K 31/4422

(54) **COMPOSITION FOR SOLID DISPERSION, SOLID DISPERSION, AND METHOD OF MANUFACTURING THE SOLID DISPERSION**

(30) Priority: 18.07.2023 JP 2023116398
(71) Applicant: Shin-Etsu Chemical Co., Ltd., Chiyoda-ku, Tokyo 1000005 (JP)
(72) Inventor: ISHIMARU, Mitsuo, YOKOHAMA-SHI, 2400005 (JP)
(74) Representative: Cabinet Nony

(57) **Abstract**

Provided is a composition for solid dispersion capable of imparting an excellent dissolution property not only under the pH conditions within the soluble pH range inherent in the acidic polymer but also under relatively low pH conditions in the upper small intestine while inhibiting decomposition of the acidic polymer.

The composition for solid dispersion contains an acidic polymer; a basic material; a poorly water-soluble drug; and an organic solvent, wherein the basic material is dispersed in the organic solvent.

## Description

### BACKGROUND OF THE INVENTION

### Field of the invention

The present invention relates to a composition for solid dispersion, a solid dispersion using the composition, and a method of manufacturing the solid dispersion. The composition contains an acidic polymer, a basic material, a poorly water-soluble drug and an organic solvent, wherein the basic material is dispersed in the organic solvent. The composition imparts an excellent dissolution property not only under the conditions within the soluble pH range of the acidic polymer but also under relatively low pH conditions in the upper small intestine.

The use of poorly water-soluble drugs has been on the rise in recent years but direct administration of such drugs does not lead to a drug exhibiting a sufficient bioavailability. For this reason, a variety of techniques for improving the solubility of such drugs has been proposed. One of such techniques is a solid dispersion method by which molecules of a poorly water-soluble drug in the form of an amorphous state are dispersed in a macromolecular carrier to enhance the apparent solubility of the poorly water-soluble drug to thereby improve the bioavailability of the drug. As a technique pertaining to the solid dispersion method, there have been proposed, for example, a spray-drying technique by which a mixture of a poorly water-soluble drug and a polymer is dissolved in a solvent, and then the mixture is dried by spraying; and a coprecipitation method by which a mixture of a poorly water-soluble drug and a polymer is dissolved in a solvent, and then the mixture undergoes precipitation.

Examples of the polymer for use as such solid dispersion include hydroxypropyl methylcellulose acetate succinate (hereinafter also referred to as "HPMCAS") which is synthesized by introducing four types, in total, of substituents-a methyl group (-CH₃), a hydroxypropyl group (-C₃H₆OH), an acetyl group (-COCH₃) and a succinyl group (-COC₂H₄COOH)- to certain hydrogen atoms within the hydroxyl groups of cellulose; and hydroxypropyl methylcellulose phthalate (hereinafter also referred to as "HPMCP") which is synthesized by introducing three types, in total, of substituents-a methyl group (-CH₃), a hydroxypropyl group (-C₃H₆OH) and a carboxybenzoyl group (-COC₆H₄COOH)- to certain hydrogen atoms within the hydroxyl groups of cellulose. These polymers (hereinafter also referred to as "acidic polymer") contain a carboxyl group in the molecule and exhibit enteric properties.

Examples of such solid dispersion using these acidic polymers include, for example, a spray-dried solid dispersion containing a poorly water-soluble drug and HPMCAS (Patent document 1).

Further, a method for manufacturing a solid dispersion using HPMCP and an oxazole compound as being a poorly water-soluble drug by spray drying can be exemplified (Patent document 2).

Furthermore, there is proposed a solid dispersion containing a neutralized acidic polymer and a poorly water-soluble drug in a form where the solubility is improved (Patent document 3).

### [Prior art documents]

### [Patent documents]

[Patent document 1] JP-A-H11-116502
[Patent document 2] JP-A-2009-502736
[Patent document 3] JP-A-2004-534822

### SUMMARY OF THE INVENTION

However, the solid dispersions of patent documents 1 and 2 contain acidic polymers, which may cause the dissolution in the upper part of the small bowel to be delayed, which often prevents sufficient improvement in bioavailability. Moreover, patent document 3 discloses that a use of naturized acidic enteric polymer can facilitate the dissolution of a drug while enhancing the concentration of the drug. However, it is necessary to add water for dissolving a basic material to be used for neutralization, which therefore makes it unfit for spray drying because the spray drying requires a poorly water-soluble drug to be dissolved.

Further, the dissolved basic material may cause a drug or an acidic polymer to be decomposed (see the working example 3 and comparative example 4 to be explained later). The main site of absorption of drugs is the small intestine, and it is desirable for the drugs to exhibit an enhanced dissolution property over a wide range of pH including a pH of 5 to 6 at the duodenum, a pH of 6 to 7 at the jejunum, and a pH of 6 to 7.5 at the ileum. Patent document 3 does not specifically disclose these effects.

The present invention has been made in view of these circumstances, and it is an object of the invention to provide a composition for solid dispersion capable of imparting an excellent dissolution property not only under the conditions within the soluble pH range inherent in the acidic polymer but also under relatively low pH conditions in the upper small intestine while inhibiting decomposition of the acidic polymer.

### [Means to solve the problems]

The inventor of the present invention diligently conducted a series of studies to solve the aforementioned problems, and completed the invention by finding that a solid dispersion having been manufactured using a composition for solid dispersion, containing an acidic polymer and a poorly water-soluble drug wherein a basic material is dispersed in an organic solvent, exhibits an excellent dissolution property not only under the conditions within the soluble pH range of the acidic polymer but also under relatively low pH conditions in the upper small intestine while such solid dispersion can also inhibit decomposition of the acidic polymer.

The present invention provides a composition for solid dispersion, a solid dispersion and a method of manufacturing the solid dispersion as defined below.
[1] A composition for solid dispersion comprising:
   an acidic polymer;
   a basic material;
   a poorly water-soluble drug; and
   an organic solvent,
   wherein the basic material is dispersed in the organic solvent.
[2] The composition according to [1], wherein the basic material is contained in an amount of 0.2 to 2.0 molar equivalents relative to the acidic groups of the acidic polymer.
[3] The composition according to [1] or [2], wherein the basic material has a volume-based average particle diameter (D50), determined by a dry laser diffractometry, of 100 µm or less.
[4] The composition according to any one of [1] to [3], wherein the acidic polymer is at least one selected from the group consisting of cellulose acetate phthalate, cellulose acetate trimellitate, cellulose acetate succinate, methylcellulose phthalate, hydroxymethyl cellulose ethyl phthalate, hydroxypropyl methylcellulose phthalate, hydroxypropyl methylcellulose acetate succinate, hydroxypropyl methylcellulose acetate maleate, hydroxypropyl methylcellulose trimellitate, carboxymethyl ethyl cellulose, polyvinyl butyrate phthalate, polyvinyl alcohol acetate phthalate, methacrylic acid/ethyl acrylate copolymer, and methacrylic acid/methyl methacrylate copolymer.
[5] The composition according to any one of [1] to [4], wherein the organic solvent is one or a mixture of two or more selected from the group consisting of acetone, methanol, ethanol, isopropanol, methyl acetate, ethyl acetate, tetrahydrofuran, and dichloromethane.
[6] A solid dispersion comprising:
   an acidic polymer;
   a basic material: and
   a poorly water-soluble drug,
   wherein the basic material has a surface that is partially or entirely coated with the acidic polymer.
[7] A method of manufacturing a solid dispersion comprising a step of removing the organic solvent from the composition as claimed in any one of [1] to [5].

According to the present invention, there can be obtained a solid dispersion that exhibits an excellent dissolution property not only under conditions within the soluble pH range of the acidic polymer but also under relatively low pH conditions in the upper small intestine.

### Brief description of the drawings

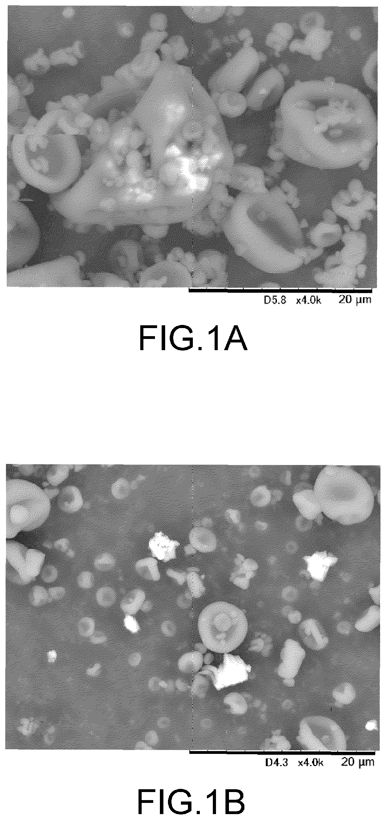

FIG. 1A is a scanning electron microscopy (SEM) image (backscattered electron image) of the solid dispersion of the working example 5. FIG. 1B is a scanning electron microscopy (SEM) image (backscattered electron image) of the solid dispersion of the comparative example 3.

### DETAILED DESCRIPTION OF THE INVENTION

### 1. Composition for solid dispersion

Described hereunder is a composition for solid dispersion containing an acidic polymer, a basic material, a poorly water-soluble drug and an organic solvent, wherein the basic material is dispersed in the organic solvent.

The term "acidic polymer" as used herein refers to an ionic polymer that has a structure containing one or more acidic functional groups, falls under the category of "practically insoluble (i.e., requiring greater than or equal to 10,000mL of water for dissolving 1 g or 1 ml of the solute)" according to the conditions as set forth in the Japanese pharmacopoeia eighteenth edition, and is soluble in an alkaline solution.

Examples of the acidic functional group include functional groups that contain carboxyl groups such as a succinyl group and a carboxybenzoyl group.

The acidic polymer enhances stability of the poorly water-soluble amorphous drug in the composition for solid dispersion and contributes to the improvement in bioavailability.

Specific examples of the acidic polymer include hydroxypropyl methylcellulose acetate succinate (hereafter also referred to as "HPMCAS"), hydroxypropyl methylcellulose phthalate (hereafter also referred to as "HPMCP"), cellulose acetate phthalate, cellulose acetate trimellitate, cellulose acetate succinate, methylcellulose phthalate, hydroxymethyl cellulose ethyl phthalate, hydroxypropyl methylcellulose acetate maleate, hydroxypropyl methylcellulose trimellitate, carboxymethyl ethyl cellulose, polyvinyl butyrate phthalate, polyvinyl alcohol acetate phthalate, methacrylic acid/ethyl acrylate copolymer (the weight ratio between the two monomers is preferably 1:99 to 99:1) and methacrylic acid/methyl methacrylate copolymer (the weight ratio between the two monomers is preferably 1:99 to 99:1).

Among them, preferred are HPMCAS and HPMCP of which HPMCAS is more preferred.

The "HPMCAS" is a cellulose derivative that is synthesized by introducing four types, in total, of substituents-an acetyl group, a succinyl group, a methyl group, and a hydroxypropyl group- to certain hydrogen atoms within the hydroxyl groups of cellulose.

HPMCAS contains acetyl groups in an amount of 2 to 16% by mass, preferably 2 to 14% by mass, more preferably 5 to 14% by mass, even more preferably 6 to 13% by mass.

HPMCAS contains succinyl groups in an amount of 4 to 28% by mass, preferably 4 to 20% by mass, more preferably 4 to 18% by mass, even more preferably 6 to 16% by mass.

It is preferred that HPMCAS contain methoxy groups in an amount of, although not limited to the followings, 12 to 28% by mass, more preferably 20 to 26% by mass.

It is preferred that HPMCAS contain hydroxypropoxy groups in an amount of, although not limited to the followings, 4 to 23% by mass, more preferably 5 to 10% by mass.

The amounts of acetyl, succinyl, methoxy, and hydroxypropyl groups contained in HPMCAS can be respectively calculated by the method as set forth in the provision of "Hypromellose acetate succinate (Hydroxypropyl methylcellulose acetate succinate)" in the Official Monographs of Japanese Pharmacopoeia 18th Edition.

The HPMCP is a cellulose derivative that contains three types of substituents-carboxybenzoyl, methoxy and hydroxypropoxy groups-in cellulose.

It is preferred that HPMCP contain carboxybenzoyl groups in an amount of 10 to 45% by mass, more preferably 15 to 40% by mass, even more preferably 21 to 35% by mass.

It is preferred that HPMCP contain methoxy groups in an amount of, although not limited to the followings, 12 to 28% by mass, more preferably 20 to 26% by mass.

It is preferred that HPMCP contain hydroxypropoxy groups in an amount of, although not limited to the followings, 4 to 23% by mass, more preferably 5 to 10% by mass.

Here, the amounts of carboxybenzoyl, methoxy and hydroxypropyl groups, contained in HPMCP, can be calculated by the method as set forth in the provision of "Hypromellose" (hydroxypropyl methylcellulose) and "Hypromellose phthalate ester" (hydroxypropyl methylcellulose phthalate) in the Official Monographs of Japanese Pharmacopoeia 18th Edition.

The acidic polymers have various soluble pH ranges (hereafter also referred to as "soluble pH") depending on the amounts of the respective substituents. For example, HPMCAS exhibits different soluble pHs depending on the amounts of acetyl and succinyl groups. Specifically, an HPMCAS containing 5 to 9% by mass of acetyl group and 14 to 18% by mass of succinyl group exhibits a soluble pH of 5.5 or more; an HPMCAS containing 7 to 11% by mass of acetyl group and 10 to less than 14% by mass of succinyl group exhibits a soluble pH of 6.0 or more; and an HPMCAS containing 10 to 14% by mass of acetyl group and 4 to 8% by mass of succinyl group exhibits a soluble pH of 6.8 or more.

Further, HPMCP exhibits different soluble pHs depending on the contents of carboxybenzoyl groups. For example, a HPMCP containing 21 to 27% by mass of carboxybenzoyl groups exhibits a soluble pH of 5.0 or more; and a HPMCP containing carboxybenzoyl groups in an amount of greater than 27% and less than or equal to 35% by mass exhibits a soluble pH of 5.5 or more.

Next, the viscosity of acidic polymers will be discussed. The following explanation specifically focuses on HPMCAS and HPMCP among the acidic polymers.

It is preferred in terms of productivity and workability that the 2% by mass HPMCAS aqueous solution (where the solvent is a 0.1mol/L diluted aqueous solution of sodium hydroxide) at 20°C exhibit a viscosity of, although not limited to the followings, 1.1 to 20 mPa·s, more preferably of 1.5 to 3.6 mPa·s.

It is preferred in terms of productivity and workability that the 10% by mass HPMCP solution (where the solvent is a solution of methanol and dichloromethane mixed to 50% of each by mass) at 20°C exhibit a viscosity of 10.0 to 150.0mPa·s, more preferably of 15.0 to 100.0mPa·s.

The viscosity of HPMCAS as an acidic polymer can be measured by the method as set forth in the provision of "hypromellose acetate succinate" in Japanese Pharmacopoeia 18th Edition. Further, the viscosity of HPMCP as an acidic polymer can be measured by the method as set forth in the provision of "Hypromellose phthalate" in Japanese Pharmacopoeia 18th Edition.

It is preferred in terms of handleability and preservation stability of the poorly water-soluble drug in the amorphous state that the amount of the acidic polymer contained in the composition for solid dispersion be 1 to 40% by mass, more preferably 1 to 10% by mass.

Two or more types of acidic polymers may be used in combination as necessary.

A commercially available acidic polymer, or an acidic polymer produced by a known method may be used.

Examples of the method for producing HPMCAS as an acidic polymer include a method in which hydroxypropyl methylcellulose is esterified with an acetylating agent and a succinylating agent under the presence of an aliphatic carboxylic acid to produce a reaction solution, and then the resultant reaction solution is mixed with water for allowing it being precipitated to prepare a suspension liquid that contains HPMCAS particles after which the HPMCAS in the suspension liquid is subjected to the treatments such as washing, collection, drying and pulverization to prepare the HPMCAS.

Examples of the method for producing HPMCP as an acidic polymer include a method in which hydroxypropyl methylcellulose is esterified with a carboxybenzoylation agent under the presence of an aliphatic carboxylic acid to produce a reaction solution, and then the resultant reaction solution is mixed with water for allowing it being precipitated to prepare a suspension liquid that contains HPMCP particles after which the HPMCP in the suspension liquid is subjected to the treatments such as washing, collection, drying and pulverization to prepare the HPMCP.

The term "basic material" refers to a material that produces a hydroxide ion when the material is in the form of a solution, and the solution exhibits basicity. The basic material is dispersed, together with the acidic polymer, in the composition for solid dispersion, and the solid dispersion is produced from the composition for solid dispersion, from which it is therefore inferred that the basic material would be dissociated simultaneously with the dissociation of the solid dispersion, and makes all or part of the acidic functional groups in the acidic polymers neutralized, which thereby contribute to the amelioration of dissolution property under relatively low pH conditions in the upper small intestine.

Examples of the basic material include hydrogencarbonates such as sodium hydrogen carbonate, potassium hydrogen carbonate and ammonium hydrogen carbonate; carbonates such as sodium carbonate, calcium carbonate, ammonium carbonate; hydroxides such as sodium hydroxide, potassium hydroxide, calcium hydroxide, magnesium hydroxide, aluminum hydroxide; ammonium salts such as ammonium hydroxide and ammonium acetate; conjugate bases with various acids such as those of sodium acetate, potassium acetate, calcium acetate, magnesium acetate, sodium citrate, trisodium phosphate, disodium phosphate, potassium citrate, sodium polyacrylate, sodium benzoate; basic amino acids such as L-arginine, L-histidine, and L-lysine; polymeric amines such as polyaminoacrylates such as Eudragit E.

It is preferred in terms of dissolution property of the solid dispersion that the basic material contained therein be in an amount of 0.2 to 2.0, more preferably 0.2 to 1.5, even more preferably 0.2 to 1.0 molar equivalents relative to the acidic groups of the acidic polymer.

It is preferred in terms of dissolution property of the solid dispersion and preventing nozzle blockage in the spray-drying process that the basic material has a volume-based average particle diameter (D50) of 100 µm or less, more preferably of 2 to 50 µm.

The volume-based average particle diameter may be determined by dry laser diffractometry. For example, as exemplified by the methods using devices such as MASTERSIZER 3000 manufactured by Malvern Panalytical Ltd in the UK and HELOS manufactured by Sympatec GmbH in Germany, a laser beam may be applied to a powder sample, sprayed by compressed air, to measure the diffraction intensity by which the volume-based average particle diameter can be calculated.

As for the volume-based average particle diameter of the basic material according to the present invention, a diameter corresponding to the 50% cumulative value of the volume-based cumulative particle diameter distribution curve was measured under conditions of a dispersion pressure of 1.5 bar and a scattering intensity of 2 to 10% by the dry method according to the Fraunhofer diffraction theory using the laser diffraction-type particle diameter distribution measuring system of Mastersizer 3000 (manufactured by Malvern Panalytical Ltd).

Two or more types of basic material may be used in combination as necessary.

Further, a basic material that is commercially available or produced by a known method may be directly used or such material subjected to a treatment such as pulverization or sieving may be used.

The term "poorly water-soluble drug" as used herein refers to a drug listed in the Japanese Pharmacopoeia Eighteenth Edition as "slightly soluble", "very slightly soluble", or "practically insoluble or insoluble" in water. The term "slightly soluble" means that when 1 g or 1 mL of a poorly water-soluble drug is placed into a beaker to which water is added and shaken vigorously at 20±5°C for 30 seconds every 5 minutes, it necessitates 100 mL or more and less than 1000 mL of water for the poorly water-soluble drug to be dissolved therein within 30 minutes. The term "very slightly soluble" means that when 1 g or 1 mL of a poorly water-soluble drug is placed into a beaker to which water is added and shaken vigorously at 20±5°C for 30 seconds every 5 minutes, it necessitates 1000 mL or more and less than 10,000 mL of water for the poorly water-soluble drug to be dissolved therein within 30 minutes. The term "practically insoluble or insoluble" means that when 1 g or 1 mL of a poorly water-soluble drug is placed into a beaker to which water is added and shaken vigorously at 20±5°C for 30 seconds every 5 minutes, it necessitates 10000 mL or more of water for the poorly water-soluble drug to be dissolved therein within 30 minutes.

Further, dissolution of a poorly water-soluble drug means that a drug dissolves or becomes miscible in a solvent, and also means that fibers, etc. are found to be un-present or present at a very slight amount when 1 g or 1 mL of the poorly water-soluble drug is placed into a beaker to which the solvent is added and shaken vigorously at 20±5°C for 30 seconds every 5 minutes.

Specific examples of the poorly water-soluble drug include azole-based compounds such as itraconazole, ketoconazole, fluconazole, miconazole, omeprazole and lansoprazole; dihydropyridine-based compounds such as nifedipine, nitrendipine, amlodipine, nicardipine, nilvadipine, felodipine and nifonidipine; propionic acid-based compounds such as ibuprofen, ketoprofen and naproxen; and indoleacetic acid-based compounds such as indomethacin and acemethacin; as well as griseofulvin; phenytoin; carbamazepine; and dipyridamole.

The amount of the poorly water-soluble drug contained therein may be suitably determined in accordance with the types of drug formulations, but it is preferred in terms of solubility and making the drug amorphous that the poorly water-soluble drug be contained in the composition for solid dispersion in an amount of 0.1 to 50% by mass, more preferably 1 to 20% by mass, and even more preferably 1 to 10% by mass.

It is preferred from the viewpoint of stability in the amorphous state of the poorly water-soluble drug that the mass ratio of the acidic polymer to the poorly water-soluble drug in the composition for solid dispersion be, although not limited to the following, expressed as follows: acidic polymer to poorly water-soluble drug = 1:0.01 to 1:100, more preferably 1:0.1 to 1:10, even more preferably 1:0.2 to 1:5.

Two or more types of poorly water-soluble drug may be used in combination as necessary.

A poorly water-soluble drug that is commercially available may also be used.

Any organic solvent may be used so long as the solvent can solubilize an acidic polymer and a poorly water-soluble drug; examples of such solvent include, but are not particularly limited to, a ketone-based solvent, an alcohol-based solvent, an ester-based solvent, an ether-based solvent and an aprotic polar solvent.

Examples of the ketone-based solvent include, for example, acetone.

Examples of the alcohol-based solvent include saturated alcohols having 1 to 3 carbon atoms such as methanol, ethanol, isopropanol.

Examples of the ester-based solvent include, for example, alkylacetates such as methyl acetate and ethyl acetate.

Examples of the ether-based solvent include, for example, tetrahydrofuran.

Examples of the aprotic polar solvent include, for example, dichloromethane and chloroform.

It is preferred in terms of productivity of the solid dispersion that the composition for solid dispersion contain an organic solvent in an amount of, although not limited to the followings, 50 to 98% by mass, more preferably 70 to 95% by mass.

One type of organic solvent may be used, or two or more types of organic solvent may be used in combination as needed. Water may be mixed into the organic solvent but it is preferred in terms of solubility of the poorly water-soluble drug and degradability of the acidic polymer that the amount of water contained therein be less than 10 parts by mass, more preferably less than 5 parts by mass per 100 parts by mass of the organic solvent.

The organic solvent may be the one that is commercially available.

The basic material in the composition for solid dispersion is dispersed in the above-mentioned organic solvent. The term "dispersed" or its associated terminology as used herein refers to a state in which all or part of the basic material is not dissolved in a solvent. The state of dispersion can be confirmed by the light transmittance obtained via UV spectrum measurement (measurement wavelength: 550 nm) of a mixture using a visible-ultraviolet spectrophotometer via cells with an optical path length of 10 mm, where the mixture can be obtained by mixing a basic material of the type same as the basic material contained in the intended composition for solid dispersion and an organic solvent of the type same as the organic solvent contained in the intended composition for solid dispersion such that the mixture has the same concentrations as the composition for solid dispersion, and then perform stirring for 30 minutes at 20°C. It is preferred that the light transmittance of the mixture be less than 90%, more preferably less than 70%, and the lower limit of the light transmittance is not particularly limited: i.e., the transmittance may be 0% or more because dispersed basic materials that are not dissolved in an organic solvent lead to a mixture having low transmittance. Specific examples of the visible-ultraviolet spectrophotometer include UV-1850 manufactured by SHIMADZU CORPORATION. When the solid dispersion is manufactured using a composition for solid dispersion in which a basic material is dispersed in an organic solvent, X-ray diffraction measurement will result in a diffraction pattern where a crystal peak originating from the basic material remains.

Further, a basic material dispersed in an organic solvent inhibits acidic functional groups in the acidic polymer from being hydrolyzed. That is, the amount of free acid produced from hydrolyzation and the associated decomposition of the drugs can be inhibited. Further, a dispersed basic material makes all or part of the acidic functional groups in the acidic polymers be neutralized simultaneously with the dissociation of the solid dispersion, which thereby contributes to the amelioration of dissolution property under relatively low pH conditions in the upper small intestine.

The composition for solid dispersion may be added with a further additive in the amount of normal use as necessary.

Examples of the additive include a dispersion stabilizer, a surfactant, a disintegrant, a binder and an extender.

Examples of the dispersion stabilizer include, for example, a talc.

Examples of the surfactant include, for example, anionic surfactants such as sodium lauryl sulfate, non-ionic surfactants such as diglycerides, poloxamers, polyoxyethylene sorbitan fatty acid esters (Tween^{®} 20, Tween^{®} 60, Tween^{®} 80), glycerol fatty acid esters, propylene glycol fatty acid esters and natural surfactants such as lecithin and sodium taurocholate.

Examples of the disintegrant include, for example, a low-substituted hydroxypropyl cellulose, cornstarch, partly pregelatinized starch, sodium carboxymethyl starch, croscarmellose, croscarmellose sodium, crystalline cellulose and crospopidone.

Examples of the binder include, for example, hydroxypropyl cellulose, polyvinylpyrrolidone, and hydroxypropyl methyl cellulose.

Examples of the extender include, for example, erythritol, mannitol, sorbitol, lactose, sucrose, starch, white sugar, crystalline cellulose, powdered cellulose, calcium phosphate and calcium sulfate.

Two or more types of additives may be used in combination as necessary. The additive may be the one that is commercially available.

It is preferred in terms of the contained amount of the poorly water-soluble drug in the solid dispersion that the contained amount of the additive in the composition for solid dispersion be 1 to 1000 parts by mass per 100 parts by mass of the total amount of the acidic polymer and the poorly water-soluble drug.

The composition for solid dispersion of the present invention can be manufactured by mixing the above-mentioned components which are the acidic polymer, the basic material, the poorly water-soluble drug, the organic solvents and, optionally, a further additive that is to be added as necessary. The mixing sequence of these components is not particularly limited but there can be exemplified a method of adding the acidic polymer and the poorly water-soluble drug into the organic solvent to obtain a solution in which the acidic polymer and the poorly water-soluble drug are dissolved, and then slowly adding the basic material to this solution and dispersing the same. The further additive that is to be added as necessary may be added thereto during a stage of preparing the solution that is initially prepared, or be added upon or after adding the basic material depending on the type of such additive.

To ensure that the composition for solid dispersion of the present invention achieves a state where the basic material is dispersed in the organic solvent, it is necessary to adjust the concentration of the basic material so that the light transmittance of the mixture of the basic material and the organic solvent, prepared under the above-specified conditions, becomes less than 90% as measured under the above-mentioned conditions. For example, if the light transmittance exceeds 90%, the amount of basic material in the composition for solid dispersion may be adjusted within the range satisfying the quantitative ratio of the acidic functional group in the acidic polymer such that the basic material maintains a high level of concentration to the extent that the basic material is not precipitated and the transmittance becomes 90% or less.

### 2. Solid dispersion

Next, there will be described a solid dispersion containing an acidic polymer, a basic material and a poorly water-soluble drug, where the surface of the basic material is partially or entirely coated with the acidic polymer. Examples of the acidic polymer, the basic material and the poorly water-soluble drug include those that are identical or similar to the examples already exemplified for explaining the composition for solid dispersion as explained above.

The basic material contained in the composition for solid dispersion of the present invention is present in a dispersed state. That is, when the solid dispersion is manufactured using this composition for solid dispersion, the basic material is entirely or partially coated with an acidic polymer. For example, in the case of using a spray drying method, the resultant droplet has a basic material in or on the droplet formed thereby, and drying of the droplet turns the droplet into a solid dispersion where the basic material is present in a mixed state around the acidic polymer and the poorly water-soluble drug. The inventor of the present invention discovered that a solid dispersion in which a basic material is partially or entirely coated with an acidic polymer significantly improves dissolution properties under relatively low pH conditions in the upper small intestine compared to an uncoated solid dispersion (i.e., a solid dispersion where the basic material is physically mixed) (See the working example 1 and the comparative example 3). The inventor has also found out that the basic material contained in a dispersed state inhibits the decomposition of the acidic polymer.

The solid dispersion, in which a basic material is partially or entirely coated with an acidic polymer, may be examined for how the basic material is coated via, for example, observation of the reflection electron image using a scanning electron microscope (SEM) by taking advantage of the distinctive elements that compose the basic material and the acidic polymer.

### 3. Manufacturing method of solid dispersion

Next, there will be described a method of manufacturing a solid dispersion comprising a step of removing the organic solvent from these compositions for solid dispersion.

Examples of the method for removing the organic solvent include evaporation drying and spray drying methods. In the present invention, it is preferable to employ the spray drying method because the method enables large-scale production and allows powder properties, such as particle diameter and volume density, to be easily controlled.

The term "spray drying" broadly refers to a method comprising the steps of breaking down a composition for solid dispersion containing a poorly soluble drug into small droplets, and rapidly removing the solvent from the droplets through evaporation. The driving force for removing the solvent can be obtained by lowering a partial pressure of the solution lower than the vapor pressure of the solvent at an ordinarily employed drying temperature of the droplets. A preferable embodiment includes, for example, a method of mixing the droplets with a high-temperature drying gas, or a method of keeping the inside of a solvent-removing apparatus at a partial vacuum.

The composition for solid dispersion can be spray-dried using a variety of nozzle mechanisms. For example, various types of nozzles can be used. For example, a two-fluid nozzle, a fountain-type nozzle, a flat fan-shaped nozzle, a pressure nozzle, or a rotary atomizer can be preferably used.

The composition for solid dispersion can be fed in the form of solution at wide ranges of flow rate and temperature. When a pressure is applied during spraying, the spraying can be carried out at a wide range of pressure. In general, as the specific surface area of droplets increases, a solvent evaporation rate increases. For this reason, at the time of release from the nozzle, it is preferred that the droplets have a size of less than 500 µm, more preferably less than 400 µm, still more preferably from 5 to 200 µm, and the flow rate, temperature and pressure enabling such spraying are preferably employed. After spraying, the solution dries rapidly.

The composition for solid dispersion dries rapidly after being sprayed, and becomes a solid dispersion. The dried solid dispersion remains in a spray drying chamber for about 5 to 60 seconds during which most of the solvent is removed from the solid powder. As the spray drying temperature, an inlet temperature is preferably from about 20°C to 150°C and an outlet temperature is preferably from 0°C to 85°C.

The solvent content remaining in the solid dispersion is preferably as small as possible. This is because the small solvent content suppresses the motility of a drug molecule in the amorphous solid dispersion and increases stability. When a further removal of the remaining solvent is required, a secondary drying can be carried out. Examples of the preferable secondary drying method include tray drying, fluidized bed drying, belt drying, and microwave drying.

### WORKING EXAMPLES

The present invention is described in detail hereunder with reference to working and comparative examples; the present invention shall not be limited to the following working examples.

Their physical properties were measured or calculated based on the methods as explained above unless otherwise stated.

In the manufacturing of the composition for solid dispersion as exemplified below, the value indicated as "% by mass" in each of the components contained in the composition for solid dispersion refers to a contained amount of the component in the composition for solid dispersion whose amount is defined as the total amount of the components.

### Working Example 1

### <Manufacturing of composition for solid dispersion >

Into 89.92% by mass of acetone were added 6.61% by mass of HPMCAS (a methoxy group content of 22.5% by mass, hydroxypropoxy group content of 6.9% by mass, acetyl group content of 7.9% by mass, succinyl group content of 14.5% by mass, 2% by mass of an aqueous solution, where the solvent is a dilute sodium hydroxide aqueous solution (0.1 mol/L) having viscosity of 2.90 mPa-s) and 3.31% by mass of nifedipine while stirring the acetone with a stirring blade to dissolve them to obtain an acetone solution. The solution was filtered through a 150 µm mesh sieve, which was confirmed that there were no undissolved components remaining therein. Sodium hydrogen carbonate having an average particle diameter (D50) of 110µm (manufactured by Fujifilm Wako Pure Chemical Corporation) was then ground three times in a Wonder Blender (manufactured by Osaka Chemical Co., Ltd.) for 10 seconds each, and then sodium hydrogen carbonate having the D50 of 8.6 µm was collected. Next, 0.16% by mass of the ground sodium hydrogen carbonate (equivalent to 0.2 molar equivalents of the succinyl group in HPMCAS) was slowly added into the acetone solution that was obtained in the above while stirring the same to disperse them to manufacture the composition A for solid dispersion in Table 2.

The ground sodium hydrogen carbonate was mixed into acetone at 20°C for 30 minutes to reach the concentration same as that of the composition for solid dispersion, and UV spectrum measurement (at a wavelength 550 nm) was conducted using a UV-visible spectrophotometer (UV-1850, manufactured by SHIMADZU CORPORATION) with a cell having 10 mm light length to measure the light transmittance.

Table 2 shows types of the acidic polymer, the basic material, the poorly water-soluble drug and the solvent, the light transmittances of the mixtures each containing the basic material and the solvent, and the additive amount and D50 of the basic material.

### <Manufacturing of solid dispersion>

The manufactured composition A for solid dispersion was spray dried using a mini spray dryer B-290 (manufactured by Nihon BUCHI K.K.) with the conditions of: inlet temperature of 80°C, outlet temperature of 52°C, nozzle gas flow rate of 360 L/hr and spraying rate of 5.7 g/min while stirring the same using a magnetic stirrer to collect the solid dispersion.

### <Crystal structure evaluation >

The crystal structure of the solid dispersion was evaluated by the XRD analysis using a benchtop XRD device D2 PHASER (manufactured by BRUKER CORPORATION). The presence or absence of the crystal peak originating from the basic material was confirmed with reference to the diffraction pattern that was obtained using the basic material only. Of all working and comparative examples, there was observed no peak originated from nifedipine, and turned out that it was amorphous. The results are as shown in Table 3.

### (XRD operating conditions)

Voltage: 30 kV
Electric current: 10 mA
Time: 0.5 s
Step: 1725
Measurement time: 1006.5 s
Measurement range 2θ: 5 to 40° (Scanning speed: 0.02)

### <Dissolution test>

The obtained solid dispersion was weighed such that it contained 90 mg (100 mg/L) of nifedipine to perform the dissolution test for 60 minutes. 900 mL of phthalate buffer solution having pH 5.0, simulating the environment in the upper small intestine, and 900 mL of the 2nd fluid having pH 6.8 for the Disintegration Test of the Japanese Pharmacopoeia 18th Edition were used as test solutions, and a Japanese Pharmacopoeia dissolution tester ("NTR-6100A" manufactured by Toyama Sangyo Co., Ltd.) was also used. The solid dispersion obtained by spray drying was in the form of extremely fine particles which were readily aggregated. For this reason, the analysis was performed with the position and rotation speed of the paddle held constant at the operating conditions as specified in Table 1.

**Table 1**

| Test period (minutes) | Rotation speed of paddle (rpm) | Position of paddle |
|---|---|---|
| 0 to 30 | 200 | 3.5 cm away from the liquid surface |
| 30 to 60 | 100 | 10 cm away from the liquid surface |

The quantity of nifedipine was determined on the basis of the absorbance measured by the UV spectrum observation (Wavelength: 325nm, optical length: 10 mm) using a concentration conversion line (calibration line) prepared from known concentration solutions. The maximum dissolution quantities into the respective test solutions are as shown in Table 3.

### <Determination of free acid amount>

The amount of free acid contained in HPMCAS refers to the total amount of the free acetic acid and the free succinic acid, which was determined by the measurement of liquid chromatography (HPLC) using the sample solution and standard solution as prepared in accordance with the procedures set forth below with the analyzing conditions as shown below. The conversion of HPMCAS into the dehydration product was calculated from a weight loss ratio when 1.0 g of a test sample was dried at 105°C for two hours. The results are as shown in Table 3.

### (Preparation of standard solution)

2.0 mL of acetic acid was precisely pipetted to which purified water was added to make it precisely a 100 mL of solution. Then, 6 mL of this solution was precisely pipetted to which water was added to make it precisely a 100 mL of acetic acid stock solution. Next, 0.13g of succinic acid was precisely weighed to which purified water was added to make it precisely a 100 mL of succinic acid stock solution. the acetic acid stock solution and succinic acid stock solution were precisely pipetted by the amounts of 4 mL each to which the mobile phase to be described below was added to make it exactly 25 mL which was served as a standard solution.

### (Preparation of sample solutions)

A solid dispersion that was manufactured to contain 100 mg of HPMCAS in the sample was accurately pipetted to which exactly 4 mL of 0.02 mol/L phosphate buffer solution of pH 7.5 was added, which was stoppered tightly and stirred for 2 hours. Then, 4 mL of diluted phosphoric acid was accurately added, which was shaken by turning the test tube upside down several times to make it mixed. It was checked if the pH of this solution had been decreased to approximately 3. If the pH was not sufficiently lowered, then a diluted phosphoric acid was further added precisely thereto and mixed by shaking. The solution was then subjected to centrifugation, and the supernatant liquid was filtered through a membrane filter (ADVANTEC^{®} manufactured by Toyo Roshi Kaisha, Ltd.) having a pore size of 0.45 µm to make it serve as a sample solution.

### (HPLC measurement conditions)

Detector: Ultraviolet absorption photometer SPD-20AV (manufactured by Shimadzu Corporation)
Measurement wavelength: 215 nm
Column: Synergi-RP (manufactured by Phenomenex Inc.)
   Inner diameter 4.6 mm, length 150 mm, particle diameter 4.0 µm,
Column temperature: 30°C
Mobile phase: 0.02 moL/L potassium dihydrogen phosphate solution (pH 2.8)
Flow rate: 1.0 mL/min
Injection volume: 10 µL

### (Conversion expression of Free acid amount)

Amount (%) of free acetic acid = (Amount (mg) of acetic acid in the standard solution/Amount (mg) of HPMCAS in the sample solution calculated on the dried basis) × (Peak area of acetic acid in the sample solution/ Peak area of acetic acid in the standard solution) × (24 × (8 + Amount (mL) of the further added diluted phosphoric acid)/2500)
Amount (%) of free succinic acid = (Amount (mg) of succinic acid in the standard solution/Amount (mg) of HPMCAS in the sample solution calculated on the dried basis) × (Peak area of succinic acid in the sample solution/ Peak area of succinic acid in the standard solution) × (4 × (8 +Amount (mL) of the further added diluted phosphoric acid)/25)
Amount of free acid (%) = Amount (%) of free acetic acid + Amount (%) of free succinic acid

### Working Example 2

An evaluation was made on a composition B for solid dispersion and its solid dispersion having been manufactured by a method similar to the method of the working example 1 except that HPMCP (a methoxy group content of 19.5% by mass; a hydroxypropyl group content of 6.1% by mass; and a carboxybenzoyl group content of 32.9% by mass, where a 10% by mass solution (methanol and dichloromethane were mixed to have the contents of 50wt% each to make it serve as a mixed solvent of the solution) had a viscosity of 42.5 mPa·s at 20°C) was used as an acidic polymer and the amount of the basic material was set as 0.5 molar equivalents relative to the carboxybenzoyl groups of HPMCP.

The types of the acidic polymer, the basic material, the poorly water-soluble drug and the solvent; the state of the basic material; the additive amount of the basic material and D50 of the basic material are as shown in Table 2.

### <Determination of free phthalic acid amount>

The amount of free acid (hereafter also referred to as "free phthalic acid amount") contained in HPMCAS was determined using the sample solution and standard solution as prepared in accordance with the procedures set forth below using the analyzing conditions as shown below. The conversion of HPMCP into the dehydration product was calculated from a weight loss ratio when 1.0 g of HPMCP was dried at 105°C for two hours. The results are as shown in Table 3.

### (Preparation of standard solution)

12.5 mg of phthalic acid was precisely weighed and 125 ml of acetonitrile was added thereto and stirred to dissolve the phthalic acid. 25 mL of purified water was then added thereinto and mixed, and acetonitrile was further added to make it exactly 250 mL which was served as a standard solution.

### (Preparation of Sample solution)

A solid dispersion that was manufactured to contain 200 mg of HPMCP in a sample was accurately pipetted, which was added into exactly 50 mL of acetonitrile and the dispersion was partially dissolved via ultrasonication. After that, 10 mL of purified water was added thereinto and ultrasonication was performed again to make it completely dissolved. The solution was cooled to the room temperature, and then acetonitrile was added thereto to make it precisely 100 mL, which was filtered through a membrane filter (ADVANTEC^{®} manufactured by Toyo Roshi Kaisha, Ltd.) having a pore size of 0.45 µm to make it serve as a sample solution.

### (HPLC measurement conditions)

Detector: Ultraviolet absorption photometer SPD-20AV (manufactured by Shimadzu Corporation)
Measurement wavelength: 235nm
Column: Luna Omega PS C18 (manufactured by Phenomenex Inc.)
   Inner diameter 4.6 mm, length 250 mm, particle diameter 5 µm
Column temperature: 25°C
Mobile phase: a mixed solution of 0.1% trifluoroacetic acid aqueous solution/acetonitrile (9:1 (volume ratio))
Flow rate: 0.5 mL/min
Injection volume: 10 µL

### (Conversion expression of free phthalic acid amount)

Amount (%) of free phthalic acid = (Amount (mg) of phthalic acid in the standard solution/Amount (mg) of HPMCP in the sample solution calculated on the dried basis) × (Peak area of phthalic acid in the sample solution/ Peak area of phthalic acid in the standard solution) × 40

### Working Example 3

An evaluation was made on a composition C for solid dispersion and its solid dispersion having been manufactured by a method similar to the method of the working example 1 except that the content of the basic material was set as 0.5 molar equivalents relative to the succinyl groups of HPMCAS.

The types of the acidic polymer, the basic material, the poorly water-soluble drug and the solvent; the state of the basic material; the additive amount of the basic material and D50 of the basic material are as shown in Table 2.

### Working Example 4

An evaluation was made on a composition D for solid dispersion and its solid dispersion having been manufactured by a method similar to the method of the working example 1 except that the content of the basic material was set as 1.0 molar equivalent relative to the succinyl groups of HPMCAS.

The types of the acidic polymer, the basic material, the poorly water-soluble drug and the solvent; the state of the basic material; the additive amount of the basic material and D50 of the basic material are as shown in Table 2.

### Working Example 5

An evaluation was made on a composition E for solid dispersion and its solid dispersion having been manufactured by a method similar to the method of the working example 1 except that calcium hydroxide (manufactured by FUJIFILM Wako Pure Chemical Corporation) having D50 of 4.3µm was used as the basic material which was contained in an amount of 0.5 molar equivalents relative to the succinyl groups of HPMCAS. Further, the reflection electron image of the obtained solid dispersion was observed using a tabletop microscope Miniscope^{®} TM3030Plus (manufactured by Hitachi High-Tech Corporation). The resultant SEM image (backscattered electron image) is as shown in Table 1A.

The types of the acidic polymer, the basic material, the poorly water-soluble drug and the solvent; the state of the basic material; the additive amount of the basic material and D50 of the basic material are as shown in Table 2.

### Working Example 6

An evaluation was made on a composition F for solid dispersion and its solid dispersion having been manufactured by a method similar to the method of the working example 5 except that the basic material was contained in an amount of 1.0 molar equivalent relative to the succinyl groups of HPMCAS.

The types of the acidic polymer, the basic material, the poorly water-soluble drug and the solvent; the state of the basic material; the additive amount of the basic material and D50 of the basic material are as shown in Table 2.

### Working Example 7

An evaluation was made on a composition G for solid dispersion and its solid dispersion having been manufactured by a method similar to the method of the working example 4 except that sodium hydrogen carbonate having D50 of 22 µm was used as the basic material.

The types of the acidic polymer, the basic material, the poorly water-soluble drug and the solvent; the state of the basic material, additive amount of the basic material and D50 of the basic material are as shown in Table 2.

### Working Example 8

An evaluation was made on a composition H for solid dispersion and its solid dispersion having been manufactured by a method similar to the method of the working example 1 except that the basic material was contained in an amount of 2.0 molar equivalent relative to the succinyl groups of HPMCAS.

The types of the acidic polymer, the basic material, the poorly water-soluble drug and the solvent; the state of the basic material; the additive amount of the basic material and D50 of the basic material are as shown in Table 2.

### Working Example 9

An evaluation was made on a composition I for solid dispersion and its solid dispersion having been manufactured by a method similar to the method of the working example 3 except that sodium hydrogen carbonate having D50 of 46 µm was used as the basic material.

The types of the acidic polymer, the basic material, the poorly water-soluble drug and the solvent; the state of the basic material; the additive amount of the basic material and D50 of the basic material are as shown in Table 2.

### Comparative Example 1

6.7% by mass of HPMCAS and 3.3% by mass of nifedipine were added into 90% by mass of acetone while stirring the acetone with a stirring blade to make it dissolve. The mixture was filtered through a sieve with an opening of 150 µm, and it was confirmed that there was no undissolved residue, and thereby a composition J for solid dispersion containing no basic material was produced. A solid dispersion was produced from the composition J for solid dispersion using a method similar to the method of the working example 1, and then the dispersion was evaluated.

The types of the acidic polymer, the poorly water-soluble drug and the solvent are as shown in Table 2.

### Comparative Example 2

An evaluation was made on a composition K for solid dispersion and its solid dispersion having been manufactured by a method similar to the method of the comparative example 1 except that the HPMCP used in the working example 2 was used as the acidic polymer. Here, the amount of free acid was determined by a method similar to the method of working example 2.

The types of the acidic polymer, the poorly water-soluble drug and the solvent are as shown in Table 2.

### Comparative Example 3

Into the solid dispersion manufactured in the comparative example 1 was added calcium hydroxide having D50 of 4.3µm as the basic material in an amount of 0.5 molar equivalents relative to the succinyl groups of HPMCAS, which was then physically mixed in a mortar. The sample in which the basic material was dispersed after manufacturing the solid dispersion was evaluated in a method similar to the method of working example 1. Further, the reflection electron image of the obtained solid dispersion was observed using a tabletop microscope Miniscope^{®} TM3030Plus (manufactured by Hitachi High-Tech Corporation) in a manner similar to the working example 5. The resultant SEM image (backscattered electron image) is as shown in Table 1B.

The types of the acidic polymer, the basic material, the poorly water-soluble drug and the solvent; the state of the basic material; the additive amount of the basic material and D50 of the basic material are as shown in Table 2.

### Comparative Example 4

An evaluation was made on a composition L for solid dispersion and its solid dispersion having been manufactured by a method similar to the method of the working example 3 except that a mixed solvent of ethanol and water that were contained at the ratio of "ethanol":"water" = 60:40 (% by mass) was used. In this example, the basic material in the composition for solid dispersion was dissolved because the solvent contained 40% by mass of water.

The types of the acidic polymer, the basic material, the poorly water-soluble drug and the solvent; the state of the basic material; the additive amount of the basic material and D50 of the basic material are as shown in Table 2.

**Table 2**

| Composition for solid dispersion | Acidic polymer | Basic material | Poorly water-soluble drug | Solvent | Light transmittance* of basic material | Added amount of basic material (molar equivalent) | D50 (µm) of basic material |
|---|---|---|---|---|---|---|---|
| A | HPMCAS | NaHCO₃ | Nifedipine | Acetone | 33 | 0.2 | 8.6 |
| B | HPMCP | NaHCO₃ | Nifedipine | Acetone | 3 | 0.5 | 8.6 |
| C | HPMCAS | NaHCO₃ | Nifedipine | Acetone | 17 | 0.5 | 8.6 |
| D | HPMCAS | NaHCO₃ | Nifedipine | Acetone | 3 | 1.0 | 8.6 |
| E | HPMCAS | Ca(OH)₂ | Nifedipine | Acetone | 3 | 0.5 | 4.3 |
| F | HPMCAS | Ca(OH)₂ | Nifedipine | Acetone | 1 | 1.0 | 4.3 |
| G | HPMCAS | NaHCO₃ | Nifedipine | Acetone | 19 | 1.0 | 22 |
| H | HPMCAS | NaHCO₃ | Nifedipine | Acetone | 1 | 2.0 | 8.6 |
| I | HPMCAS | NaHCO₃ | Nifedipine | Acetone | 65 | 0.5 | 46 |
| J | HPMCAS | none | Nifedipine | Acetone | - | 0.0 | - |
| K | HPMCP | none | Nifedipine | Acetone | - | 0.0 | - |
| L | HPMCAS | NaHCO₃ | Nifedipine | Ethanol/wa ter = 6/4 | 100 | 0.5 | 86 |

| | | | | | | | |
|---|---|---|---|---|---|---|---|
| *Light transmittance of the mixture in which the basic material was dispersed or dissolved in the solvent (Observed wavelength 550 nm) | | | | | | | |

**Table 3**

| | Used composition for solid dispersion | Crystal peak of basic material | Maximum dissolution quantity (mg/L) | | Amount of free acid (%) |
|---|---|---|---|---|---|
| | | | pH 5 0 | pH 6 8 | |
| Working example 1 | A | Present | 58 | 93 | 0.024 |
| Working example 2 | B | Present | 45 | 60 | 0.664 |
| Working example 3 | C | Present | 67 | 96 | 0.023 |
| Working example 4 | D | Present | 67 | 91 | 0.028 |
| Working example 5 | E | Present | 60 | 93 | 0.043 |
| Working example 6 | F | Present | 63 | 92 | 0.122 |
| Working example 7 | G | Present | 66 | 93 | 0.027 |
| Working example 8 | H | Present | 66 | 86 | 0.029 |
| Working example 9 | I | Present | 56 | 96 | 0.024 |
| Comparative example 1 | J | Absent | 42 | 96 | 0.031 |
| Comparative example 2 | K | Absent | 33 | 63 | 0.600 |
| Comparative example 3 | J* | Present | 47 | 93 | 0.031 |
| Comparative example 4 | L | Absent | 62 | 89 | 0.115 |

| | | | | | |
|---|---|---|---|---|---|
| * *Magnesium hydroxide having D50 of 4.3 µm was further added in an amount of 0.5 molar equivalents relative to succinyl groups of HPMCAS* | | | | | |

It has been found out from the results of the working examples 1 to 2 and the comparative examples 1 to 2 that a composition for solid dispersion containing an acidic polymer, a basic material, a poorly water-soluble drug and an organic solvent can significantly improve dissolution property of nifedipine in a test solution of pH 5.0 which simulate the pH in the upper small intestine. Further, a crystal peak of the basic material was observed in the diffraction pattern obtained by the XRD measurement in all of the working examples, which therefore demonstrates that the basic material was in a dispersed state in the composition for solid dispersion.

It has also been found out from the results of the working example 3 and comparative example 4 that the decomposition of an acidic polymer can be inhibited by using acetone as a solvent and making the basic material in a dispersed state.

It has also been found out from the results of working example 5 and comparative example 3 that a solid dispersion having been manufactured by dispersing the basic material in the composition for solid dispersion has an enhanced dissolution property in a test solution of pH 5.0 compared with the one in which the basic material is physically mixed after manufacturing the solid dispersion. Moreover, it was shown from comparison between the backscattered electron SEM images that the basic material, having been turned into a solid dispersion in a dispersed state, allows the surface of the basic material to be partially or entirely coated with an acidic polymer. This can be read out from fact that calcium elements having large atomic number are observed as bright (white) since such elements reflect an increased emission signal intensity of irradiated electrons during the SEM observation. It is therefore inferred that this coating enhances the miscibility between the acidic polymer and the basic material.

It has also been found out from the results of the working examples 1, 3, 4 and 8 that the solid dispersion having been manufactured using the composition exhibits an excellent dissolution property in the test solutions of pHs 5.0 and 6.8 in a wide range of basic material content of 0.2 to 2.0 molar equivalents relative to the acidic groups in the acidic polymer.

## Claims

1. A composition for solid dispersion comprising:
an acidic polymer;
a basic material;
a poorly water-soluble drug; and
an organic solvent,
wherein the basic material is dispersed in the organic solvent.

2. The composition according to claim 1, wherein the basic material is contained in an amount of 0.2 to 2.0 molar equivalents relative to the acidic groups of the acidic polymer.

3. The composition according to claim 1, wherein the basic material has a volume-based average particle diameter (D50), determined by a dry laser diffractometry, of 100 µm or less.

4. The composition according to claim 1, wherein the acidic polymer is at least one selected from the group consisting of cellulose acetate phthalate, cellulose acetate trimellitate, cellulose acetate succinate, methylcellulose phthalate, hydroxymethyl cellulose ethyl phthalate, hydroxypropyl methylcellulose phthalate, hydroxypropyl methylcellulose acetate succinate, hydroxypropyl methylcellulose acetate maleate, hydroxypropyl methylcellulose trimellitate, carboxymethyl ethyl cellulose, polyvinyl butyrate phthalate, polyvinyl alcohol acetate phthalate, methacrylic acid/ethyl acrylate copolymer, and methacrylic acid/methyl methacrylate copolymer.

5. The composition according to claim 1, wherein the organic solvent is one or a mixture of two or more selected from the group consisting of acetone, methanol, ethanol, isopropanol, methyl acetate, ethyl acetate, tetrahydrofuran, and dichloromethane.

6. A solid dispersion comprising:
an acidic polymer;
a basic material: and
a poorly water-soluble drug,
wherein the basic material has a surface that is partially or entirely coated with the acidic polymer.

7. A method of manufacturing a solid dispersion comprising a step of removing the organic solvent from the composition as claimed in any one of claims 1 to 5.
